# FASCICULE DE BREVET EUROPEEN

(11) **EP 0 889 969 B1**
(45) Date de publication et mention de la délivrance du brevet: **09.11.2005**
(21) Numéro de dépôt: 97914375.7
(22) Date de dépôt: 12.03.1997
(51) Int. Cl.: C12N 15/86, C07K 14/705, A61K 48/00, C12N 5/10, A61K 39/00, C12N 15/12

(54) **VECTEURS ADENOVIRAUX RECOMBINANTS POUR LA THERAPIE GENIQUE DES TUMEURS HUMAINES**
REKOMBINANTE ADENOVIRENVEKTOREN ZUR GENTHERAPIE DER MENSCHLICHEN KREBSE
RECOMBINANT ADENOVIRAL VECTORS FOR HUMAN TUMOUR GENE THERAPY

(30) Priorité: 14.03.1996 FR 9603207
(43) Date de publication de la demande: 13.01.1999
(73) Titulaire: Aventis Pharma S.A., 92160 Antony (FR); LUDWIG INSTITUTE FOR CANCER RESEARCH, London SW1E 5AG (GB)
(72) Inventeur: BOON, Falleur, B-1050 Bruxelles (BE); DUFFOUR, Marie-Thérèse, F-75005 Paris (FR); HADDADA, Hedi, F-94140 Alfortville (FR); LURQUIN, Christophe, B-1950 Kraainem (BE); PERRICAUDET, Michel, F-28320 Ecrosnes (FR); UYTTENHOVEGHESQUIERE, Catherine, B-1325 Chaumont-Gistoux (BE); WARNIER, Guy, B-1630 Linkebeek (BE)
(74) Mandataire: Lecca, Patricia S.
(86) Numéro de dépôt international: PCT/FR1997/000435
(87) Numéro de publication internationale: WO 1997/034009

(56) Documents cités:
- WO-A-92/20356
- WO-A-94/21126
- WO-A-94/23031
- WO-A-95/02697
- JOURNAL OF IMMUNOLOGY, vol. 156, no. 1, 1 Janvier 1996, BALTIMORE US, pages 224-231, XP000674674 CHEN, P.W. ET AL.: "Therapeutic antitumor response after immunization with a recombinant adenovirus encoding a model tumor-associated antigen"
- SCIENCE, vol. 254, 13 Décembre 1991, LANCASTER, PA US, pages 1643-1647, XP000604921 VAN DEN BRUGEN, P. ET AL.: "A gene encoding an antigen recognized by cytolytic T lymphocytes on a human melanoma" cité dans la demande
- INTERNATIONAL JOURNAL OF CANCER, vol. 67, no. 2, 17 Juillet 1996, pages 303-310, XP000675638 WARNIER, G. ET AL.: "Induction of a cytolytic T-cell response in mice with a recombinant adenovirus coding for tumor antigen P815"

### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96
   (i) DEPOSANT:
      (A) NOM: LUDWIG INSTITUTE FOR CANCER RESEARCH
      (B) RUE: 1345, avenue of the Americas
      (C) VILLE: NEW YORK
      (E) PAYS: ETATS-UNIS
      (F) CODE POSTAL: 10105
   (ii) TITRE DE L' INVENTION: METHODE DE TRAITEMENT PAR THERAPIE GENIQUE DES TUMEURS HUMAINES ET VIRUS RECOMBINANTS CORRESPONDANTS.
   (iii) NOMBRE DE SEQUENCES: 3
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 126 paires de bases
      (B) TYPE: nucléotide
      (C) NOMBRE DE BRINS: double
      (D) CONFIGURATION: linéaire
   (ii) TYPE DE MOLECULE: ADNc
   (xi) DESCRIPTION DE LA SEQUENCE: SEQ ID NO: 1:
   TTGAATTCGC CGCCATGGAG TCCTTGCAGC TGGTCTTTGG CATTGACGTG     50
   AAGGAAGCAG ACCCCACCGG CCACTCCTAT GTCCTTGTCA CCTGCCTAGG    100
   TCTCTCCTAT GATGGCTAGA ATTCTT                              126
(2) INFORMATIONS POUR LA SEQ ID NO: 2:
   (i) CARACTERISTIQUES DE LA SEQUENCE:
      (A) LONGUEUR: 44 paires de bases
      (B) TYPE: nucléotide
      (C) NOMBRE DE BRINS: double
      (D) CONFIGURATION: linéaire
   (ii) TYPE DE MOLECULE: ADNc
   (xi) DESCRIPTION DE LA SEQUENCE: SEQ ID NO: 2:
   AATTCGCCGC CATGGAAGTG GACCCCATCG GCCACTTGTA CTAG     44
(2) INFORMATIONS POUR LA SEQ ID NO: 3:
   (i) CARACTERISTIQUES DE LA SEQUENCE:
      (A) LONGUEUR: 44 paires de bases
      (B) TYPE: nucléotide
      (C) NOMBRE DE BRINS: double
      (D) CONFIGURATION: linéaire
   (ii) TYPE DE MOLECULE: ADNc
   (xi) DESCRIPTION DE LA SEQUENCE: SEQ ID NO: 3:
   AATTCGCCGC CATGCTGCCT TATCTAGGGT GGCTGGTCTT CTAG          44

## Description

La présente invention concerne une méthode de traitement, des tumeurs humaines par la thérapie génique. Elle concerne en particulier l'utilisation des virus recombinants défectifs portant une séquence codant pour un antigène spécifique des tumeurs humaines, pour le traitement préventif ou curatif des tumeurs humaines ainsi que pour générer in vitro ou ex vivo des CTL spécifiques. Elle concerne également des compositions pharmaceutiques comportant ces virus, notamment sous forme injectable.

La thérapie génique consiste à corriger une déficience ou une anomalie en introduisant une information génétique dans la cellule ou l'organe affecté. Cette information peut être introduite soit in vitro dans une cellule extraite de l'organe et ensuite réinjectée dans l'organisme, soit in vivo, directement dans le tissu visé. S'agissant d'une molécule de haut poids moléculaire et de charge négative, l'ADN a des difficultés pour traverser spontanément les membranes cellulaires phospholipidiques. Différents vecteurs sont donc utilisés afin de permettre le transfert de gène : des vecteurs viraux d'une part, des vecteurs chimiques etlou biochimiques, naturels ou synthétiques, d'autre part. Les vecteurs chimiques et/ou biochimiques sont par exemple des cations (phosphate de calcium, DEAE-dextran,...) qui agissent en formant des précipités avec l'ADN, lesquels peuvent être "phagocytés" par les cellules. Il peut également s'agir de liposomes dans lesquels l'ADN est incorporé et qui fusionnent avec la membrane plasmique. Les vecteurs synthétiques de transfert de gènes sont généralement des lipides ou des polymères cationiques qui complexent l'ADN et forment avec lui une particule portant des charges positives en surface. Ces particules sont capables d'interagir avec les charges négatives de la membrane cellulaire, puis de franchir celle-ci. On peut citer comme exemples de tels vecteurs le dioctadécylamidoglycylspermine (DOGS, Transfectam™) ou le chlorure de N-[1-(2,3-dioleyloxy)propyl]-N,N,N-triméthylammonium (DOTMA, Lipofectin™). Des protéines chimères ont aussi été développées : elles sont constituées d'une partie polycationique qui condense l'ADN, liée à un ligand qui se fixe sur un récepteur membranaire et entraîne le complexe dans les cellules par endocytose. Il est ainsi théoriquement possible de "cibler" un tissu ou certaines populations cellulaires, afin d'améliorer la biodisponibilité in vivo du gène transféré (pour revues, voir Behr, 1993, Cotten et Wagner, 1993). Parmi les virus potentiellement utilisables comme vecteurs pour le transfert de gènes on peut citer plus particulièrement les rétrovirus (RSV, HMS, MMS, etc.), le virus HSV, les virus adéno-associés, et les adénovirus. Ces virus ont tous été utilisés pour infecter différents types cellulaires.

Des approches de thérapie génique ont été développées pour le traitement de différents types de pathologies, incluant les désordres du système nerveux, les maladies cardiovasculaires ou les cancers. Concernant plus particulièrement le domaine des cancers, différentes approches ont été proposées dans l'art antérieur. Ainsi, des études décrivent l'utilisation de lymphocytes activés ex vivo par culture en présence d'interleukine-2 ou par transfection avec le gène de l'interleukine-2. Des études d'immunothérapie adoptive ont également été entreprises avec des monocytes-macrophages purifiés et activés ex vivo par de l'interféron pour augmenter leur pouvoir tumoricide, puis réinjectés aux patients (Andressen et al., Cancer Res. 50 (1990) 7450). La possibilité d'utiliser des macrophages génétiquernent modifiés a également été décrite (WO95/06120). Une autre série d'approches est basée sur le transfert de gènes toxiques capables d'induire de manière directe ou indirecte la mort des cellules cancéreuses. Ce type d'approche a été décrit par exemple avec le gène de la thymidine kinase, transféré in vivo soit par un vecteur adénoviral (PCT/FR94/01284; PCT/FR94/01285), soit par greffe de cellules productrices d'un vecteur rétroviral (Caruso et al., PNAS 90 (1993) 7024). D'autres gènes utilisés sont par exemple le gène de la cytosine désaminase.

Le présente demande est relative à une nouvelle méthode de traitement des cancers. Elle est tout particulièrement destinée au traitement des mélanomes. La méthode de l'invention repose sur le transfert et l'expression in vivo d'antigènes spécifiques des mélanomes, capables d'induire (i) une protection immunitaire contre l'apparition de ce type de cancers et (ii) une expansion de la population de cellules T cytotoxiques (CTL) spécifiques des cellules présentant ces antigènes et ainsi une destruction par le système immunitaire des cellules tumorales correspondantes.

Le système immunitaire a entre autres fonctions la capacité d'assurer une protection contre les infections virales. Cette capacité est assurée par des lymphocytes T cytotoxiques (CTL). Les CTL présentent deux caractéristiques remarquables : ils sont hautement spécifiques et d'une grande efficacité. Ils détruisent les cellules infectées après avoir repéré un antigène viral à leur surface. L'antigène en question se manifeste sous la forme d'un peptide associé à une molécule du complexe majeur d'histocompatibilité de classe I (CMH-I). Dans le cadre des tumeurs, il a été observé, initialement chez la souris, que ces cellules malignes présentent des complexes peptide-molécules du CMH-I capables de provoquer, comme dans le cadre des réponses antivirales, une réponse immunitaire médiée par des CTL. Ces peptides proviennent notamment de protéines codées par des gènes mutés ou activés sélectivement dans les cellules tumorales. Ces protéines sont désignés antigènes spécifiques de tumeurs. Plus récemment, des antigènes de différenciation reconnus par des CTL ont été caractérisés sur des tumeurs humaines.

La présente invention concerne une nouvelle méthode de traitement des tumeurs humaines. Elle découle en particulier de la mise au point de vecteurs d'origine virale capables de transférer et d'exprimer in vivo des antigènes spécifiques de tumeurs humaines ou mélanomes. Elle repose plus particulièrement sur la démonstration dans les modèles murins que les adénovirus recombinants défectifs sont capables d'induire une immunisation contre ce type d'antigènes, permettant d'obtenir in vivo des réponses lymphocytaires contre ces antigènes et notamment les cellules tumorales les portant. Cette méthode selon l'invention permet donc par le transfert de ces gènes d'agir sur l'évolution des tumeurs humaines de manière particulièrement efficace en stoppant leur progression, pouvant conduire à l'éradication.

La présente invention décrit donc un adénovirus recombinant défectif contenant, inséré dans son génome, un acide nucléique codant pour une protéine ou un peptide spécifique de tumeur et plus particulièrement pour tout ou partie d'un antigène spécifique d'un mélanome.

Préférentiellement, il s'agit d'un antigène spécifique d'un mélanome humain. Encore plus préférentiellement, il s'agit d'un fragment d'un antigène spécifique d'un mélanome humain comprenant la partie présentée aux CTL en association avec les molécules du CMH-I. Les antigènes spécifiques de tumeurs humaines ont été décrits par Thierry Boon et al. (US5,342,774; US5,405,940; WO92/20356; WO94/23031; WO94/21126). Ces antigènes, désignés sous le terme de MAGE, sont exprimés sélectivement dans les cellules tumorales, principalement les tumeurs humaines. Différents gènes MAGE humains ont été décrits, et notamment les gènes MAGE-1, MAGE-2, MAGE-3, MAGE-4, MAGE-5, MAGE-6, MAGE-7, MAGE-8, MAGE-9, MAGE-10, MAGE-11, MAGE-12. A titre représentatif de gènes murins homologues on peut également faire état des gènes S-MAGE-1 et S-MAGE-2. En ce qui concerne plus particulièrement des gènes BAGE, GAGE et RAGE, ils sont représentatifs d'autres familles de gènes apparentés.

Dans la cadre de la présente invention est décrit un adénovirus recombinant défectif contenant, inséré dans son génome, un acide nucléique codant pour une protéine ou peptide dérivant de celle-ci sélectionnée parmi les protéines Mage-1, Mage-3, Bage, Gage et Rage. Ces antigènes sont en effet les plus sélectifs, en ce sens qu'ils n'ont été détectés pour la plus grande majorité sur aucune cellule somatique non tumorale. La séquence de l'antigène mage-1 et du gène correspondant ont été décrites notamment dans Van der Bruggen et al., Science 254 (1991) 1644). La séquence de l'ADNc codant pour Mage-1 et Mage-3 a été décrite par exemple dans Gaugler et al. (J. Exp. Med. 179 (1994) 921).

Comme indiqué ci-avant, il est décrit un adénovirus recombinant défectif contenant, inséré dans son génome, un acide nucléique codant pour un peptide de la protéine Mage -1, Mage-3, Bage ou Gage comprenant la partie présentée : aux CTL en association avec les molécules du CMH-I. Les gènes Mage, Bage et Gage codent en effet pour des protéines de taille importante. Ces protéines sont dégradées par digestion enzymatique dans la cellule, conduisant à la génération de peptides. Ce sont ces peptides qui sont alors présentés à la surface des cellules, et qui sont reconnus par les CTL en association avec les molécules du CMH-I (Cf figure 2). Encore plus préférentiellement, l'invention concerne un adénovirus recombinant comprenant, inséré dans son génome, un acide nucléique codant pour un peptide de la protéine Mage-1 ou Mage-3 comprenant la partie présentée aux CTL.

Selon un mode de réalisation spécifique, il est décrit un adénovirus recombinant comprenant, inséré dans son génome, la séquence SEQ ID n° 1. Cette séquence comprend la séquence codant pour le nonapeptide (27pb) de Mage-1 qui est présenté par la molécule HLA.A1 aux lymphocytes T cytotoxiques. Encore plus préférentiellement, il s'agit de la séquence comprise entre les résidus 55 à 82 de la séquence SEQ ID n°1.

Selon un autre mode de réalisation spécifique, il est décrit un adénovirus recombinant comprenant, inséré dans son génome, la séquence SEQ ID n° 2. Cette séquence comprend la séquence codant pour le nonapeptide (27pb) de Mage-3 qui est présenté par la molécule HLA.A1 aux lymphocytes T cytotoxiques.

Selon un autre mode de réalisation, il est décrit un adénovirus recombinant comprenant, inséré dans son génome, un acide nucléique codant pour le peptide antigénique du gène P1A du mastocytome p815 de la souris DBA/2 (SEQ ID n° 3).

Comme indiqué précédemment, les adénovirus permettent un transfert et une expression efficace de ces peptides antigéniques in vivo. Ainsi, ils permettent de manière tout à fait remarquable, de stimuler in vivo l'apparition de lymphocytes T cytotoxiques spécifiques de ces antigènes, qui détruisent sélectivement toute cellule présentant à sa surface cet antigène.

Les virus décrits dans le cadre de l'invention sont donc utilisables pour la préparation de compositions pharmaceutiques destinées au traitement des cancers dont les cellules présentent des antigènes Mage à leur surface. Pour préparer de telles compositions, les cellules tumorales (généralement d'un mélanome) d'un patient sont préférentiellement prélevées et analysées pour (i) déterminer l'expression d'un gène Mage par exemple, par RT-PCR, et (ii) le cas échéant, typer cet antigène Mage. Un adénovirus contenant un acide nucléique codant pour tout ou partie de l'antigène correspondant est construit et utilisé pour l'administration.

Les virus peuvent également être utilisés in vitro (ou ex vivo), pour générer des populations de cellules T cytotoxiques spécifiques d'un antigène tumoral donné. Pour cela, une population de cellules est infectée par un virus de l'invention, puis mise en contact avec des précurseur de cellules CTL. Les cellules CTL spécifiques des antigènes peuvent alors être sélectionnées in vitro, amplifiées, puis utilisées à titre de médicament pour détruire spécifiquement les tumeurs correspondantes. Avantageusement, la population de cellules infectée par un virus de l'invention comprend des cellules presentatrices d'antigènes (APC). Il peut s'agir en particulier de macrophages (WO95/06120) ou de cellules B.

Dans les adénovirus décrits dans le cadre de l'invention, l'acide nucléique inséré peut être un fragment d'ADN complémentaire (ADNc), d'ADN génomique (ADNg), ou une construction hybride consistant par exemple en un ADNc dans lequel seraient insérés un ou plusieurs introns. Il peut également s'agir de séquences synthétiques ou semisynthétiques. Comme indiqué ci-avant, il s'agit d'un acide nucléique codant pour tout une protéine ou peptide derivant de cette protéine sélectionnée parmi Mage-1, Mage-3, Bage et Gage. Au sens de la présente invention, l'expression peptide derivant de cette protéine signifie que l'acide nucléique peut coder pour un fragment seulement de la protéine, ce fragment devant être susceptible de générer des CTL. Le fragment selon l'invention porte donc au moins un déterminant antigénique reconnu par un CTL spécifique. Ces fragments peuvent être obtenus par toute technique connue de l'homme du métier, et notamment par modifications génétique et/ou chimique et/ou enzymatique, ou encore par clonage par expression, permettant la sélection de variants en fonction de leur activité biologique. Les modifications génétiques incluent les suppressions, délétions, mutations, etc.

L'acide nucléique inséré est préférentiellement un ADNc ou d'un ADNg.

Généralement, l'acide nucléique inséré comprend également des séquences permettant l'expression dans la cellule infectée de l'antigène ou du fragment d'antigène. Il peut s'agir des séquences qui sont naturellement responsables de l'expression dudit antigène lorsque ces séquences sont susceptibles de fonctionner dans la cellule infectée. Il peut également s'agir de séquences d'origine différente, désignées séquences hétérologues (responsables de l'expression d'autres protéines, ou même synthétiques). Notamment, il peut s'agir de promoteurs de gènes eucaryotes ou viraux ou de séquences dérivées, stimulant ou réprimant la transcription d'un gène de façon spécifique ou non et de façon inductible ou non. A titre d'exemple, il peut s'agir de séquences promotrices issues du génome de la cellule que l'on désire infecter, ou du génome d'un virus, et notamment, les promoteurs des gènes E1A, MLP d'adénovirus, le promoteur CMV, LTR-RSV, SRα, etc. Parmi les promoteurs eucaryotes, on peut citer également les promoteurs ubiquitaires (HPRT, vimentine, α-actine, tubuline, etc), les promoteurs des filaments intermédiaires (desmine, neurofilaments, kératine, GFAP, etc) les promoteurs de gènes thérapeutiques (type MDR, CFTR, facteur VIII, etc) les promoteurs spécifiques de tissus (pyruvate kinase, villine, promoteur de la protéine intestinale de liaison des acides gras, promoteur de l'actine α des cellules du muscle lisse, promoteurs spécifiques pour le foie ; Apo Al, Apo All, Albumine humaine etc) ou encore les promoteurs répondant à un stimulus (récepteur des hormones stéroïdes, récepteur de l'acide rétinoïque, etc,). En outre, ces séquences d'expression peuvent être modifiées par addition de séquences d'activation, de régulation, etc. Par ailleurs, lorsque l'acide nucléique inséré ne comporte pas de séquences d'expression, il peut être inséré dans le génome du virus défectif en aval d'une telle séquence.

Les virus utilisés dans le cadre de la présente invention sont défectifs, c'est-à-dire incapables de se répliquer de façon autonome dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par le gène inséré. Préférentiellement, le virus défectif conserve néanmoins les séquences de son génome qui sont nécessaires à l'encapsidation des particules virales.

Les virus utilisés dans le cadre de l'invention peuvent être obtenus à partir de différents sérotypes d'adénovirus. Il existe différents sérotypes d'adénovirus, dont la structure et les propriétés varient quelque peu. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mavl, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs utilisés dans le cadre de l'invention comprenent les ITR, une séquence permettant l'encapsidation et l'acide nucléique d'intérêt. Encore plus préférentiellement, dans le génome des adénovirus utilisés dans le cadre de l'invention, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697) et L5 (WO95/02697). Selon un mode préféré de mise en oeuvre, l'adénovirus utilisés dans le cadre de l'invention comprend une délétion dans les régions E1 et E4. Selon un autre mode de réalisation préféré, il comprend une délétion dans région E1 au niveau de laquelle sont insérés la région E4 et l'acide nucléique (Cf FR94 13355). Avantageusement, la délétion dans la région E1 porte sur les nucléotides 454 à 3328 (fragment PvuII-BgIII) ou 382. à 3446 (fragment HinfII-Sau3A). Avantageusement, la délétion dans la région E4 comprend au moins les phases ORF3 et ORF6.

L'acide nucléique d'intérêt peut être inséré en différentes régions du génome de l'adénovirus. Le génome d'un adénovirus est composé d'un ADN double brin linéaire d'une taille de 36 kb environ. Il comprend notamment une séquence inversée répétée (ITR) à chaque extrémité, une séquence d'encapsidation (Psi), des gènes précoces et des gènes tardifs (Cf figure 1). Les principaux gènes précoces sont contenus dans les régions E1, E2, E3 et E4. Parmi ceux-ci, les gènes contenus dans la région E1 sont nécessaires à la propagation virale. Les principaux gènes tardifs sont contenus dans les régions L1 à L5. Le génome de l'adénovirus Ad5 a été entièrement séquencé et est accessible sur base de données (voir notamment Genebank M73260). De même des parties, voire la totalité d'autres génomes d'adénoviraux (Ad2, Ad7, Ad12, etc.) ont également été séquencées. L'acide nucléique d'intérêt est préférentiellement inséré dans une région non essentielle à la production des virus recombinants défectifs. Ainsi, il est préférentiellement inséré au niveau de la région E1, qui est défective dans le virus et complémentée par la lignée de production, de la région E3, qui n'est pas essentielle à la production des virus recombinants (son inactivation ne doit pas être transcomplémentée), ou encore dans la région E4. Dans ce dernier cas, il est nécessaire de complémenter les fonctions E4 lors de la production, soit par co-transfection avec un plasmide ou virus helper, soit au moyen d'une lignée appropriée. Il est clair que d'autres sites peuvent être utilisés. En particulier, l'accès à la séquence nucléotidique du génome permet à l'homme du métier d'identifier des régions permettant d'insérer l'acide nucléique d'intérêt.

Les adénovirus recombinants défectifs utilisés dans le cadre de l'invention peuvent être préparés par toute technique connue de l'homme du métier (Levrero et al., Gene 101 (1991) 195, EP 185 573; Graham, EMBO J. 3 (1984) 2917). Généralement, les adénovirus sont produits par transfection de l'ADN du virus recombinant dans une lignée cellulaire d'encapsidation compétente. Il peut s'agir d'une transfection simple, lorsque l'on peut disposer d'une construction portant l'ensemble du génome du virus recombinant, ou, comme c'est le cas le plus souvent, d'une co-transfection de plusieurs fragments d'ADN apportant les différentes parties du génome viral recombinant. Dans ce cas, le procédé implique une ou plusieurs étapes de recombinaison homologue entre les différentes constructions dans la lignée cellulaire d'encapsidation, pour générer l'ADN du virus recombinant. Les différents fragments utilisés pour la production du virus peuvent être préparés de différentes façons. La technique la plus généralement utilisée consiste à isoler l'ADN viral puis à le modifier in vitro par les méthodes classiques de biologie moléculaire (digestion, ligation, etc.). Les constructions obtenues sont ensuite purifiées et utilisées pour transfecter les lignées d'encapsidation. Une autre technique repose sur l'utilisation d'un plasmide portant une partie du gènome du virus recombinant, qui est co-transfecté avec un virus apportant la partie manquante du gènome. Une autre possibilité réside dans l'utilisation de plasmides procaryotes pour préparer les ADN viraux utilisables pour la transfection (Cf Bett et al., PNAS 91 (1994) 8802; FR95 01632).

La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697.

Ensuite, les adénovirus qui se sont multipliés sont récupérés et purifiés selon les techniques classiques de biologie moléculaire, comme illustré dans les exemples.

La présente invention décrit également toute composition pharmaceutique comprenant un ou plusieurs adénovirus recombinants défectif tel que décrit ci-avant. Les compositions pharmaceutiques de l'invention peuvent être formulées en vue d'une administration par voie orale, parentérale, intranasale, intraveineuse, intramusculaire, sous-cutanée, transdermique, intra-trachéale, intra-péritonéale, etc.

La présente invention concerne également toute composition pharmaceutique comprenant des cellules infectées par un adénovirus recombinant défectif tel que décrit ci-avant. Avantageusement, la composition de l'invention comprend des cellules présentatrices d'antigènes (APC) infectées par un adénovirus recombinant défectif tel que décrit ci-avant. A titre d'exemple particulier, on peut citer les macrophages ou les lymphocytes B. L'invention concerne encore une composition comprenant des cellules T cytotoxiques (CTL) spécifiques d'un antigène tumoral, préparées par culture de cellules précurseurs en présence de cellules présentatrices d'antigènes (APC) infectées par un adénovirus recombinant défectif tel que décrit ci-avant.

Préférentiellement, une composition pharmaceutique de l'invention contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution de virus, et est déterminé par infection d'une culture cellulaire appropriée, et mesure, généralement après 15 jours, du nombre de plages de cellules infectées. Les techniques de détermination du titre pfu d'une solution virale sont bien documentées dans la littérature.

Selon l'antigène concerné, les adénovirus de l'invention peuvent être utilisés pour le traitement ou la prévention de cancers, incluant notamment les tumeurs humaines (pour les antigènes Mage-1 à Mage 12, Gage et Bage et Rage) les sarcomes (pour les antigènes Mage-1).

La présente invention sera plus complètement décrite à l'aide des exemples qui suivent, qui doivent être considérés comme illustratifs et non limitatifs.

### Techniques générales de clonage, de biologie moléculaire.

Les méthodes classiques de biologie moléculaire telles que la centrifugation d'ADN plasmidique en gradient de chlorure de césium-bromure d'éthidium, les digestions par les enzymes de restriction, l'électrophorèse sur gel,la transformation dans E.coli, la précipitation des acides nucléiques etc, sont décrites dans la littérature (Maniatis et al., 1989).

Les enzymes ont été fournies par New-England Biolabs (Beverly, MA).

Pour les ligatures les fragments d'ADN sont séparés selon leur taille sur des gels d'agarose de 0,8 à 1,5 %, purifiés par GeneClean (BIO101, LaJolla CA) et incubés de nuit à 14°C dans un tampon Tris-HCl pH 7.4 50 mM, MgCl₂ 10 mM, DTT 10 mM, ATP 2 mM, en présence d'ADN ligase du phage T4.

L'amplification par PCR, (Polymerase Chain Reaction), a également été réalisée selon Maniatis et al., 1989, avec les spécifications suivantes :
- Concentration en MgCl₂ portée à 8mM.
- Température de dénaturation 95°C, température d'hybridation 55°C, température d'allongement 72°C. Ce cycle a été répété 25 fois dans un PE9600 Thermalcycler (Perkin Elmer, Norwalk CO).

Les oligonucléotides sont synthétisés en utilisant la chimie des phosphoramidites protégés en β par un groupement cyanoéthyl, (Sinha et al.,1984, Giles 1985), avec le synthétiseur automatique d'ADN Applied Biosystem modèle 394, (Applied Biosystem, Foster City CA), selon les recommandations du fabricant.

Le séquençage a été effectué sur des matrices double-brin par la méthode de terminaison de chaînes en utilisant des amorces fluorescentes. Nous avons utilisé le kit se séquencage Taq Dye Primer Kit de chez Applied Biosystem (Applied Biosystem, Foster City CA) selon les spécifications du fabricant.

### Exemple 1 : Construction d'un adénovirus recombinant défectif codant pour un fragment d'antigène P1A.

Cet exemple décrit la construction d'un adénovirus recombinant défectif selon l'invention codant pour un fragment de l'antigène P1A. Plus particulièrement, l'adénovirus porte la séquence SEQ ID n°3. L'adénovirus construit est un adénovirus de sérotype 5, possédant une délétion dans les régions E1 et E3, l'acide nucléique d'intérêt étant inséré dans la région E1, au niveau de la délétion.

L'acide nucléique inséré au niveau de la région E1 comprend plus
- le promoteur SRα. Le promoteur SRα comprend l'origine de réplication précoce de SV40 et une partie du LTR du HTLV1 (correspondant au domaine R et à une partie de U5), suivis de la jonction d'épissage 16S de SV40 (Takebe et al., Mol. Cell. Biol. 8 (1988) 466).
- un minigène P1A de 44 pb (SEQ ID N° 3).
- le site de polyadénylation du virus SV40.

Cet acide nucléique a été extrait du plasmide pcD-SRα-P1A, correspondant au plasmide pcD-SRα dans lequel le minigène P1A a été cloné au site EcoRI. L'insert obtenu a ensuite été cloné dans le plasmide pAd.RSV-βGal (Stratford-Perricaudet et al., J. Clin. Invest. 90 (1992) 626), à la place du fragment comportant le LTR de RSV et le gène LacZ.

Le plasmide pAd-SRα-P1A ainsi obtenu a ensuite été utilisé pour produire l'adénovirus recombinant. Pour cela, les cellules de la lignée 293 ont été co-transfectées par 5 µg de plasmide pAd-SRα-P1A et par 5 µg de l'ADN de l'adénovirus mutant dl 324 en présence de phosphate de calcium. Les adénovirus recombinants produits ont ensuite été sélectionnés par purification sur plaque. Après isolement, l'adénovirus recombinant est amplifié dans la lignée cellulaire 293, ce qui conduit à un surnageant de culture contenant l'adénovirus recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'analyse de l'ADN viral par digestion à l'aide d'enzymes de restriction EcoRI démontre la présence de l'insert dans le génome. L'adénovirus peut être conservé à - 80°C dans 10 % de glycérol.

### Exemple 2 : Construction d'un adénovirus recombinant défectif codant pour un fragment d'antigène Mage-1.

Cet exemple décrit la construction d'un adénovirus recombinant défectif selon l'invention codant pour un fragment de l'antigène Mage-1. Plus particulièrement, l'adénovirus porte la séquence SEQ ID n°1 codant pour un fragment portant le nonapeptide antigénique de Mage-1. L'adénovirus construit est un adénovirus de sérotype 5, possédant une délétion dans les régions E1 et E3, l'acide nucléique d'intérêt étant inséré dans la région E1, au niveau de la délétion.

L'acide nucléique inséré au niveau de la région E1 comprend plus particulièrement :
- le promoteur SRα. Le promoteur SRα comprend l'origine de réplication précoce de SV40 et une partie du LTR du HTLV1 (correspondant au domaine R et à une partie de U5), suivis de la jonction d'épissage 16S de SV40 (Takebe et al., Mol. Cell. Biol. 8 (1988) 466).
- un minigène MAGE-1 de 116 pb (SEQ ID N° 1). Ce fragment a été obtenu par PCR à partir du gène Mage-1 complet. Il comporte un ATG en position 15, un codon stop en position 121 et une partie de l'exon 3 du gène Mage-1. Il comprend la séquence correspondant au nonapeptide (27pb) qui est présenté par la molécule HLA.A1 aux lymphocytes T cytotoxiques.
- le site de polyadénylation du virus SV40.

Cet acide nucléique a été extrait du plasmide pcD-SRα-MAGE-1, correspondant au plasmide pcD-SRα dans lequel le minigène Mage-1 a été cloné au site EcoRI. L'insert obtenu a ensuite été cloné dans le plasmide pAd.RSV-βGal (Stratford-Perricaudet et al., J. Clin. Invest. 90 (1992) 626), à la place du fragment comportant le LTR de RSV et le gène LacZ.

Le plasmide pAd-SRα-MAGE-1 ainsi obtenu a ensuite été utilisé pour produire l'adénovirus recombinant. Pour cela, les cellules de la lignée 293 ont été co-transfectées par le plasmide pAd-SRα-MAGE-1 et par l'ADN de l'adénovirus mutant dl 324 en présence de phosphate de calcium. Les adénovirus recombinants produits ont ensuite été sélectionnés par purification sur plaque. Après isolement, l'adénovirus recombinant est amplifié dans la lignée cellulaire 293, ce qui conduit à un surnageant de culture contenant l'adénovirus recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'analyse de l'ADN viral par digestion à l'aide d'enzymes de restriction EcoRI démontre la présence de l'insert dans le génome. L'adénovirus peut être conservé à - 80°C dans 10 % de glycérol.

### Exemple 3 : Construction d'un adénovirus recombinant défectif codant pour un fragment d'antigène Mage-3.

Cet exemple décrit la construction d'un adénovirus recombinant défectif selon l'invention codant pour un fragment de l'antigène Mage-3. Plus particulièrement, l'adénovirus porte la séquence SEQ ID n°2 codant pour un fragment portant le nonapeptide antigénique de Mage-3. L'adénovirus construit est un adénovirus de sérotype 5, possédant une délétion dans les régions E1 et E3, l'acide nucléique d'intérêt étant inséré dans la région E1, au niveau de la délétion.

L'acide nucléique inséré au niveau de la région E1 comprend plus particulièrement :
le promoteur SRα. Le promoteur SRα comprend l'origine de réplication précoce de SV40 et une partie du LTR du HTLV1 (correspondant au domaine R et à une partie de U5), suivis de la jonction d'épissage 16S de SV40 (Takebe et al., Mol. Cell. Biol. 8 (1988) 466).
   - un minigène MAGE-3 de 44 pb (SEQ ID N° 2). Ce fragment a été obtenu par PCR à partir du gène Mage-3 complet. Il comporte un ATG en position 12, un codon stop en position 44 et la séquence correspondant au nonapeptide (27pb) qui est présenté par la molécule HLA.A1 aux lymphocytes T cytotoxiques.
   - le site de polyadénylation du virus SV40.

Cet acide nucléique a été extrait du plasmide pcD-SRα-MAGE-3, correspondant au plasmide pcD-SRα dans lequel le minigène Mage-3 a été cloné au site EcoRI. L'insert obtenu a ensuite été cloné dans le plasmide pAd.RSV-βGal, à la place du fragment comportant le LTR de RSV et le gène LacZ.

Le plasmide pAd-SRα-MAGE-3 ainsi obtenu a ensuite été utilisé pour produire l'adénovirus recombinant. Pour cela, les cellules de la lignée 293 ont été co-transfectées par le plasmide pAd-SRα-MAGE-3 et par l'ADN de l'adénovirus mutant dl324 en présence de phosphate de calcium. Les adénovirus recombinants produits ont ensuite été sélectionnés par purification sur plaque. Après isolement, l'adénovirus recombinant est amplifié dans la lignée cellulaire 293, ce qui conduit à un surnageant de culture contenant l'adénovirus recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'analyse de l'ADN viral par digestion à l'aide d'enzymes de restriction EcoRI démontre la présence de l'insert dans le génome. L'adénovirus peut être conservé à - 80°C dans 20 % de glycérol.

### Exemple 4 : Caractérisation fonctionnelle des adénovirus de l'invention

Cet exemple démontre que les virus selon l'invention sont capables d'induire l'expression du gène d'intérêt codant pour une protéine dont la dégradation conduit à l'expression d'un peptide antigénique à la surface des cellules cibles.

L'expression du minigène MAGE-1 et la présentation du peptide ont été mises en évidence sur des cellules infectées par l'Ad-Mage (4.1.), par détermination de la lyse spécifique (4.2.) et stimulation de la production de TNF (4.3.).

### 4.1. Lignées cellulaires

Les lignées cellulaires qui ont été infectées sont les suivantes :
- C1R.A1 : lignée lymphocytaire B transformée par l'EBV (réf. Storkus, W.J., Howell, D. N., Salter, R. D., Dawson, J. R., and Cresswell, P. : NK susceptibility varies inversety with target cell class I HLA antigen expression. J. Immunol. 138 : 1675 - 1659, 1987) et transfectée par le gène HLA.A1 cloné dans le plasmide pHEBO.
- Gerl III β E⁻F⁻ : Cellules de mélanome humain HLA.A1 immunosélectionnées pour la perte de l'antigène MAGE-1. (désignées Gerlach E⁻ dans les tableaux 1 et 2).

Ces deux lignées expriment donc la molécule HLA.A1 mais pas l'antigène MAGE-1.

### 4.2. Détermination de la lyse spécifique

Cet exemple démontre l'existence d'une lyse spécifique des cellules par un clone de CTL spécifique de l'antigène (test de relarguage du chrome radioactif). Pour cela, les cellules mentionnées en 4.1. ont été infectées par l'adénovirus Ad-Mage1 (exemple 2) ou par un adénovirus contrôle (Ad-βGal) à une multiplicité d'infection de 500 pfu/cellule. Les cellules infectées ont ensuite été marquées au chrome 51 puis incubées pendant 4 heures, à raison de 1000 cellules / puits, avec le CTL spécifique (clone 82:30) à différents rapports cellules effectrices/cellules cibles (E/T). Le pourcentage de lyse a ensuite été déterminé. Les résultats obtenus sont présentés dans le tableau 1. Ils montrent clairement que les cellules infectées par les virus selon l'invention présentent une sensibilité à la lyse par les CTL spécifiques nettement supérieure à celle des cellules infectées par l'adénovirus contrôle. Ces cellules présentent également une sensibilité nettement accrue par rapport aux cellules directement transfectées par l'antigène Mage-1, ce qui démontre l'efficacité thérapeutique des vecteurs de l'invention.

Ces résultats montrent donc clairement que les virus de l'invention sont capables de conférer à des cellules une sensibilité importante à la lyse par les CTL spécifiques.

### 4.3. Stimulation de la production de TNF

Dans cet exemple, la capacité des cellules Gerl III β E⁻F⁻ à stimuler la production de TNF par le même clone de CTL a été évaluée. Pour cela, les cellules Gerl III β E⁻F⁻ ont été infectées par l'adénovirus Ad-Mage1 (exemple 2) ou par un adénovirus contrôle (Ad-βGal) à une multiplicité d'infection de 50 ou 100 pfu/cellule, puis incubées avec le clone CTL. Après 24 heures, la quantité de TNF présente dans les surnageants a été mesurée par détermination de leur cytotoxicité sur une lignée sensible au TNF (lignée WEHI-164-13). La viabilité cellulaire a été mesurée au moyen d'un test colorimétrique (MTT). Les résultats sont exprimés en densité optique puis en quantité de TNF (pg/ml). Ce test n'a pas été effectué sur les cellules C1 R.A1 car elles sécrétent naturellement du TNF. Les cellules contrôles Gerlach E⁺ sont des cellules de mélanome exprimant Mage - 1 et HLA - A1.

Les résultats obtenus sont présentés dans le tableau 2. Ils montrent clairement que les cellules infectées par les virus selon l'invention induisent une production de TNF par les CTL, ce qui confirme sans ambiguité les propriétés biologiques et thérapeutiques des virus de l'invention.

### Exemple 5 : Activité in vivo des virus PIA de l'invention

L'exemple 4 a montré que, in vitro ou ex vivo, les cellules infectées par un adénovirus selon l'invention sont bien reconnues en test TNF et lysées en test de relarguage de chrome radioactif par un CTL spécifique.

Cet exemple démontre maintenant que in vivo, les adénovirus selon l'invention sont capables de générer une réponse CTL spécifique. Plus particulièrement, cet exemple démontre que 2 injections à une semaine d'intervalle de 10⁹ particules virales (pfu) dans les souris DBA/2 génèrent chez une partie d'entre elles une forte réponse CTL spécifique.

Deux séries d'expériences ont été réalisées. Dans la première série d'expériences, les particules virales ont été administrées pour moitié dans la cavité péritonéale et pour l'autre moitié sous la peau (en 4 sites). Dans la deuxième série d'expériences, les particules virales ont été administrées par injections sous-cutanées, intra-péritonéales, intra-nasales et intra-trachéales. Ces deux séries d'expériences ont été réalisées selon le protocole suivant. Les souris ont été sacrifiées 15 jours après la seconde série d'injections de l'adénovirus. Les splénocytes de ces souris ont ensuite été mis en présence de l'antigène P815A au moyen de cellules L1210A+ (leucémie syngénique transfectée avec le gène P₁A du mastocytome P815) irradiées pour une période de 8 jours. Au terme de cette culture mixte tumeur-lymphocytes (MLTC), les lymphocytes dirigés contre l'antigène P815A ont proliféré et se sont différenciés en lymphocytes T tueurs. Ceux-ci sont alors mis en présence de cellules marquées avec du chrome radioactif. Il s'agit de cellules P511, un variant azaguanine - résistant du mastocytome de souris P815 portant l'antigène A et de PI.204, un variant de ce même mastocytome ayant perdu l'antigène A. Ce dernier sert de contrôle négatif. Afin d'éliminer toute possibilité de réaction non spécifique, les cellules cibles marquées avec du chrome radioactif sont également mises en présence de cellules syngéniques (L1210) portant à leur surface les antigènes de la cellule stimulante dans la MLTC à l'exception de l'antigène A de P815.

Les résultats obtenus avec la première série d'expérience, exprimés en pourcentage de lyse spécifiques sont présentés dans les tableaux 3A et 3B.

Les résultats obtenus avec la deuxième série d'expérience, exprimés en pourcentage de lyse spécifiques, sont présentés dans les tableaux 4A et 4B.

Dans les deux cas, les résultats présentés montrent, souris par souris, des niveaux de lyse proportionnels aux rapports effecteurs/cibles (moyennes de duplicats). Des CTL ont été obtenus quel que soit le site d'injection de l'adénovirus (Tableau 4A + B). Ces résultats montrent clairement que les adénovirus de l'invention sont capables de générer in vivo une immunité contre les cellules porteuses de l'antigène tumoral.

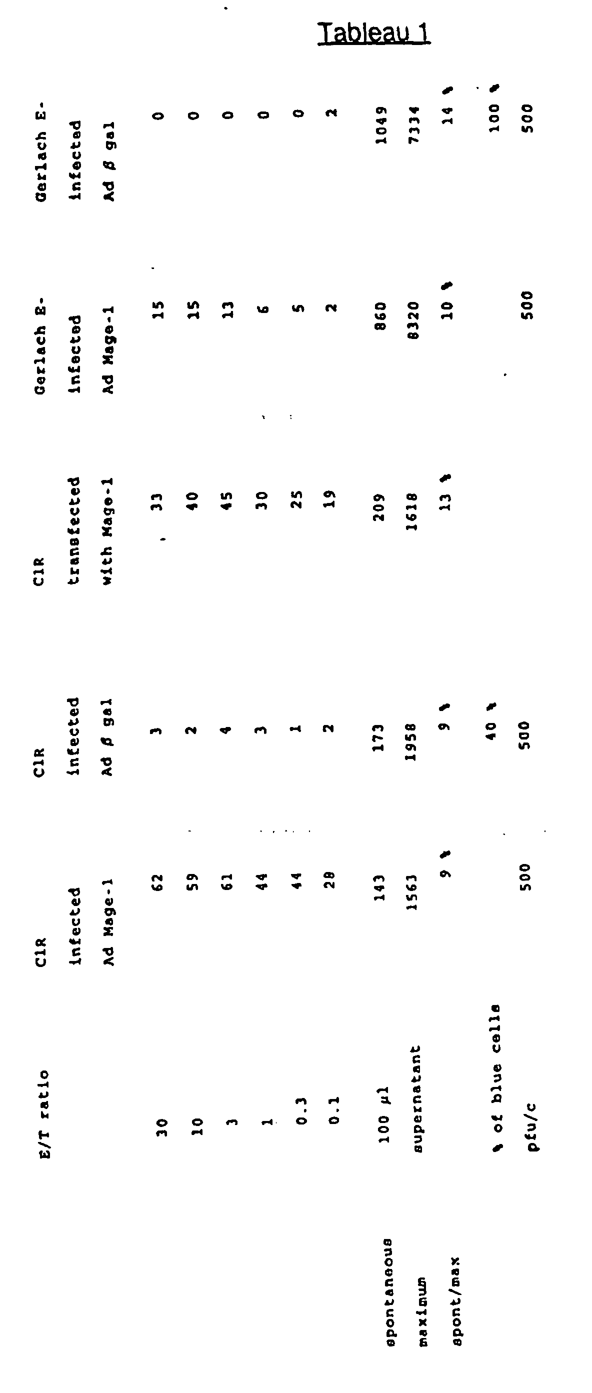

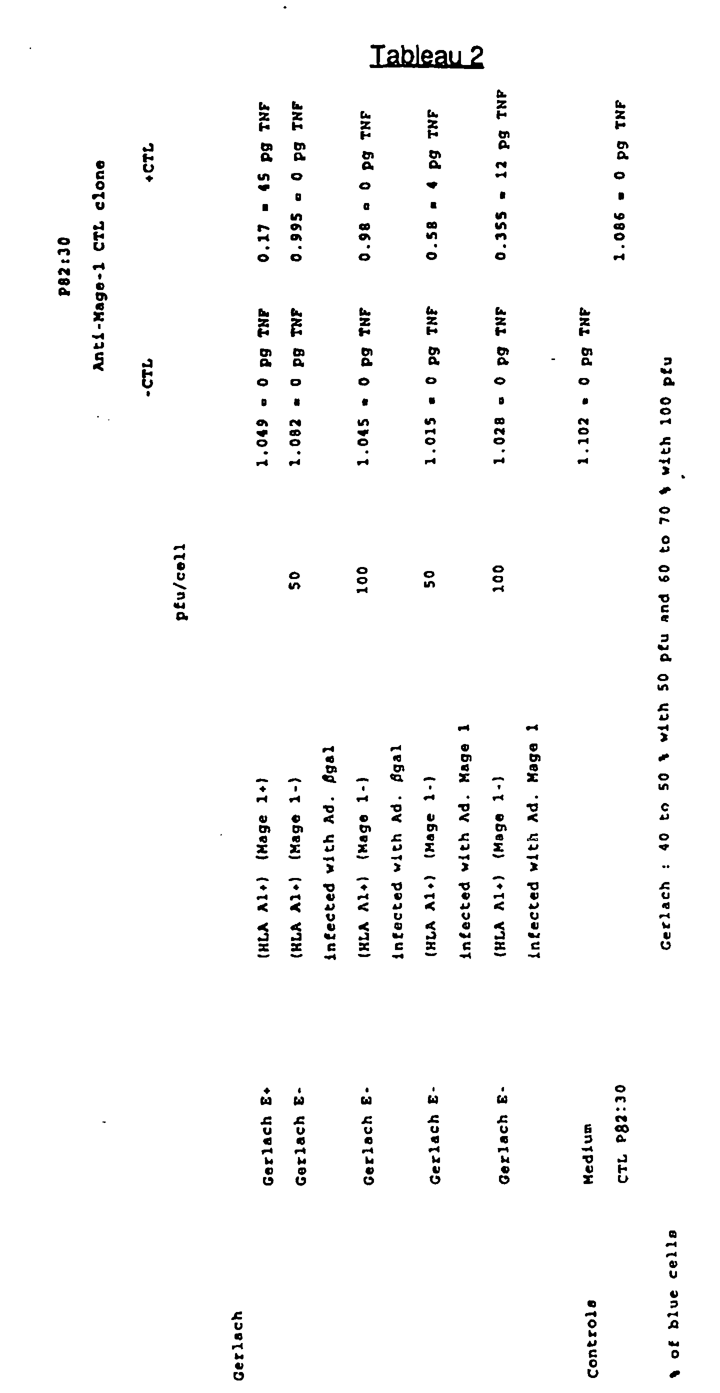

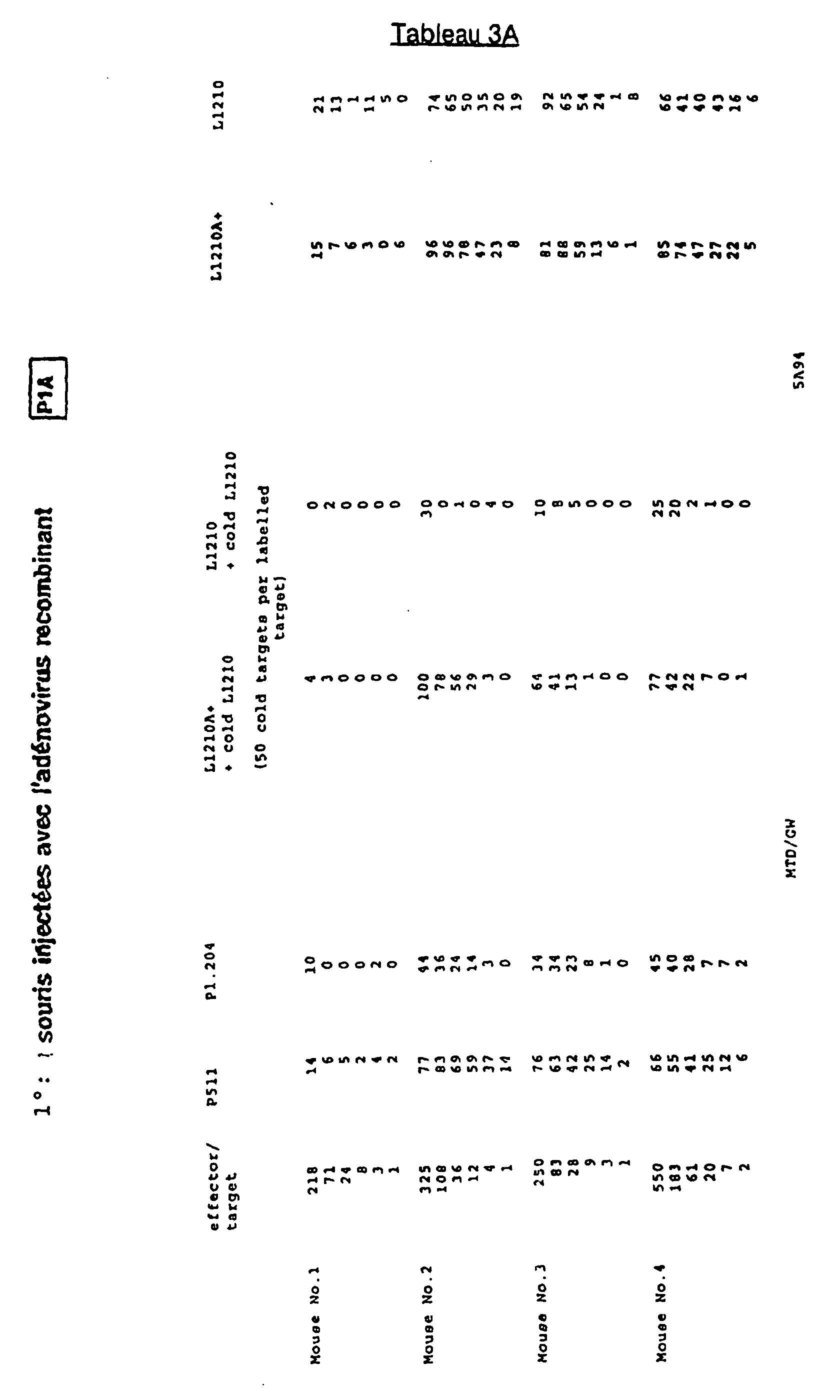

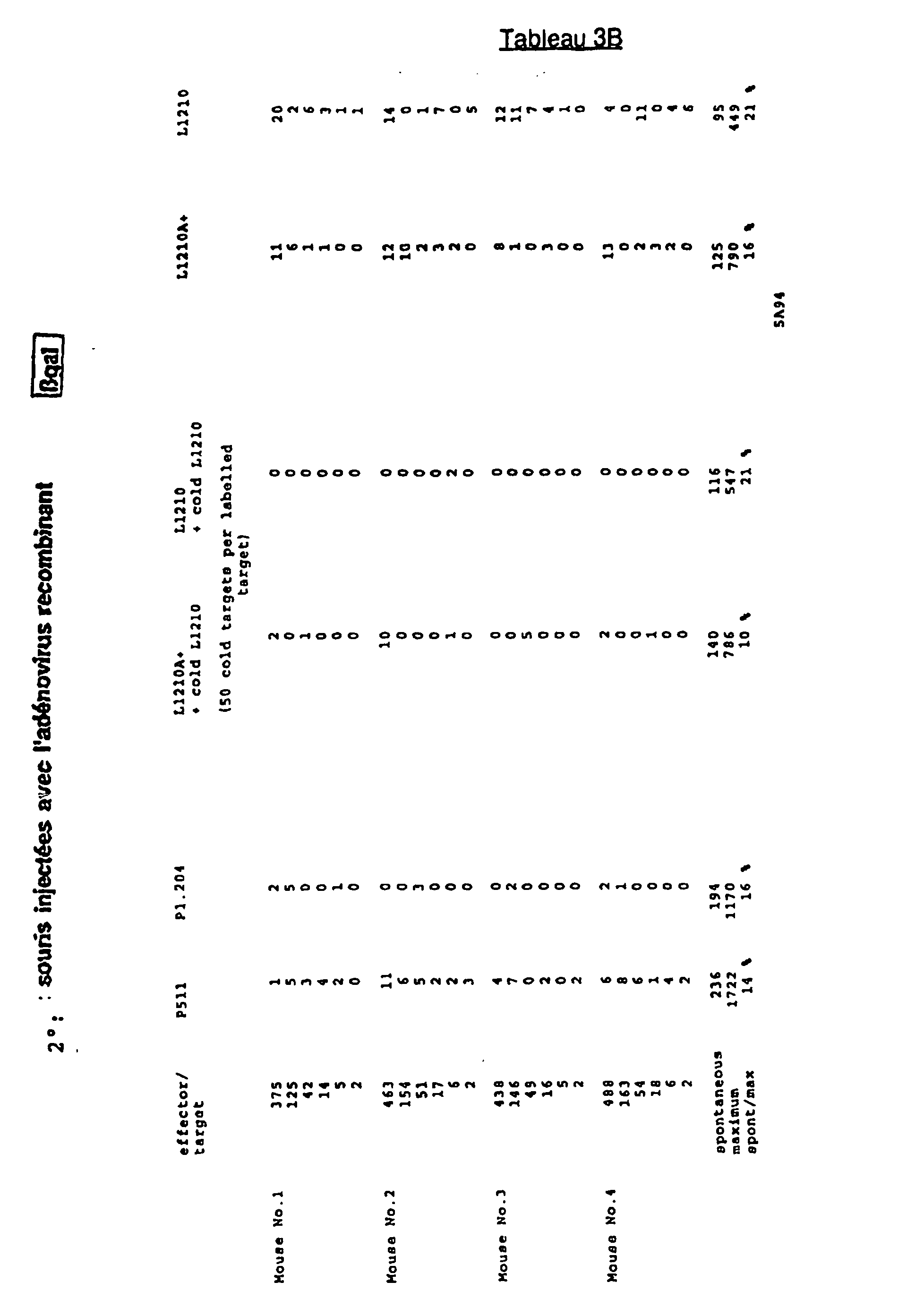

**Tableau 4A**

| | | Effector/ target | P511 | P1.204 | P511 + cold L1210 50><1 ratio | P1.204 + cold L1210 50><1 ratio |
|---|---|---|---|---|---|---|
| Subcutaneous | | | | | | |
| | Mouse No.1 | 490 | 9 | 2 | 0 | 1 |
| | | 163 | 5 | 3 | 0 | 0 |
| | | 54 | 3 | 1 | 0 | 0 |
| | | 18 | 3 | 0 | 0 | 0 |
| | | 8 | 2 | 1 | 0 | 0 |
| | | 2 | 0 | 0 | 0 | 0 |
| | | | | | | |
| | Mouse No.2 | 50 | 5 | 0 | 0 | 1 |
| | | 27 | 3 | 0 | 0 | 0 |
| | | 9 | 5 | 0 | 3 | 0 |
| | | 3 | 6 | 0 | 0 | 0 |
| | | 1 | 4 | 0 | 1 | 0 |
| | | 0.3 | 2 | 0 | 0 | 0 |
| | | | | | | |
| | Mouse No.3 | 300 | 73 | 18 | 68 | 0 |
| | | 100 | 71 | 15 | 72 | 0 |
| | | 33 | 92 | 10 | 75 | 1 |
| | | 11 | 73 | 8 | 57 | 8 |
| | | 4 | 56 | 3 | 25 | 0 |
| | | 1 | 25 | 2 | 8 | 0 |

| Intraperitoneal | | | | | | |
|---|---|---|---|---|---|---|
| | Mouse No.1 | 258 | 51 | 9 | 48 | 0 |
| | | 85 | 43 | 10 | 37 | 0 |
| | | 28 | 43 | 8 | 27 | 0 |
| | | 9 | 38 | 3 | 12 | 1 |
| | | 3 | 18 | 2 | 8 | 0 |
| | | 1 | 7 | 0 | 1 | 2 |
| | | | | | | |
| | Mouse No.2 | 295 | 9 | 4 | 9 | 2 |
| | | 88 | 8 | 6 | 2 | 0 |
| | | 33 | 7 | 4 | 2 | 0 |
| | | 11 | 3 | 2 | 1 | 0 |
| | | 4 | 1 | 2 | 0 | 0 |
| | | 1 | 0 | 0 | 0 | 1 |
| | | | | | | |
| | Mouse No.3 | 400 | 68 | 12 | 76 | 0 |
| | | 133 | 77 | 12 | 82 | 0 |
| | | 44 | 88 | 14 | 73 | 0 |
| | | 15 | 14 | 7 | 66 | 0 |
| | | 6 | 10 | 6 | 30 | 0 |
| | | 3 | 10 | 1 | 14 | 0 |
| | | | | | | |
| | Mouse No.4 | 185 | 8 | 2 | 3 | 0 |
| | | 52 | 3 | 0 | 3 | 0 |
| | | 57 | 10 | 2 | 3 | 0 |
| | | 8 | 4 | 1 | 2 | 0 |
| | | 2 | 3 | 0 | 0 | 0 |
| | | 0.8 | 2 | 0 | 2 | 0 |

| Intratracheal | | | | | | |
|---|---|---|---|---|---|---|
| | Mouse No.1 | 440 | 67 | 11 | 50 | 3 |
| | | 147 | 78 | 13 | 52 | 2 |
| | | 49 | 64 | 6 | 49 | 0 |
| | | 18 | 34 | 8 | 23 | 0 |
| | | 6 | 31 | 7 | 9 | 0 |
| | | 2 | 14 | 0 | 2 | 0 |
| | | | | | | |
| | Mouse No.2 | 410 | 8 | 7 | 3 | 2 |
| | | 137 | 6 | 7 | 3 | 1 |
| | | 45 | 6 | 4 | 0 | 1 |
| | | 15 | 5 | 2 | 2 | 0 |
| | | 9 | 1 | 3 | 0 | 0 |
| | | 2 | 1 | 2 | 0 | 2 |
| | | | | | | |
| | Mouse No.3 | 344 | 8 | 2 | 3 | 1 |
| | | 115 | 4 | 0 | 2 | 0 |
| | | 38 | 8 | 0 | 1 | 0 |
| | | 13 | 8 | 0 | 0 | 0 |
| | | 4 | 4 | 1 | 0 | 0 |
| | | 1 | 1 | 0 | 0 | 0 |
| | | | | | | |
| | Mouse No.4 | 265 | 80 | 9 | 52 | 0 |
| | | 85 | 46 | 8 | 47 | 0 |
| | | 28 | 42 | 7 | 21 | 0 |
| | | 9 | 33 | 3 | 9 | 0 |
| | | 3 | 17 | 2 | 2 | 0 |
| | | 1 | 5 | 2 | 1 | 0 |
| | | | | | | |
| | Mouse No.5 | 230 | 2 | 0 | 1 | 0 |
| | | 77 | 4 | 0 | 3 | 0 |
| | | 25 | 2 | 0 | 0 | 0 |
| | | 8 | 2 | 0 | 0 | 0 |
| | | 3 | 1 | 1 | 0 | 0 |
| | | 1 | 1 | 2 | 0 | 0 |
| | | | | | | |
| | Mouse No.6 | 210 | 13 | 2 | 11 | 0 |
| | | 70 | 10 | 0 | 7 | 0 |
| | | 23 | 6 | 0 | 2 | 1 |
| | | 3 | 1 | 0 | 0 | 0 |
| | | 3 | 2 | 0 | 0 | 0 |
| | | 1 | 2 | 0 | 0 | 0 |

| Intranasal | | | | | | |
|---|---|---|---|---|---|---|
| | Mouse No.1 | 430 | 58 | 0 | 52 | 6 |
| | | 160 | 52 | 0 | 58 | 1 |
| | | 53 | 84 | 4 | 65 | 0 |
| | | 18 | 84 | 2 | 52 | 0 |
| | | 6 | 51 | 2 | 33 | 0 |
| | | 2 | 52 | 0 | 20 | 0 |
| | | | | | | |
| | Mouse No.2 | 205 | 4 | 0 | 0 | 2 |
| | | 69 | 4 | 0 | 0 | 0 |
| | | 83 | 1 | 0 | 0 | 0 |
| | | 8 | 5 | 0 | 0 | 0 |
| | | 2 | 3 | 0 | 0 | 0 |
| | | 0.8 | 2 | 0 | 0 | 0 |
| | Mouse No.3 | 250 | 1 | 0 | 0 | 0 |
| | | 83 | 0 | 0 | 0 | 0 |
| | | 28 | 1 | 0 | 0 | 0 |
| | | 9 | 1 | 0 | 0 | 0 |
| | | 3 | 1 | 0 | 0 | 0 |
| | | 1 | 1 | 0 | 0 | 0 |
| | | | | | | |
| | Mouse No.4 | 280 | 2 | 0 | 1 | 0 |
| | | 87 | 8 | 1 | 0 | 0 |
| | | 29 | 1 | 0 | 0 | 0 |
| | | 10 | 2 | 0 | 1 | 0 |
| | | 3 | 0 | 0 | 0 | 0 |
| | | 1 | 1 | 1 | 0 | 0 |
| | | | | | | |
| | Mouse No.5 | 240 | 3 | 0 | 0 | 2 |
| | | 80 | 1 | 0 | 0 | 0 |
| | | 27 | 1 | 0 | 0 | 1 |
| | | 9 | 1 | 1 | 0 | 0 |
| | | 3 | 1 | 0 | 0 | 0 |
| | | 1 | 1 | 1 | 1 | 0 |
| | | | | | | |
| | Mouse No. 6 | 125 | 0 | 0 | 0 | 0 |
| | | 42 | 0 | 0 | 0 | 0 |
| | | 14 | 0 | 0 | 0 | 0 |
| | | 5 | 1 | 0 | 0 | 0 |
| | | 2 | 0 | 0 | 0 | 1 |
| | | 0.6 | 0 | 0 | 0 | 0 |

**Tableau 4B**

| | | | | | | |
|---|---|---|---|---|---|---|
| 2°: Adβgal injected mice | | | | | | |

| | | Effector/ target | P511 | P1.204 | P511 + cold L1210 50><1 ratio | P1.204 + cold L1210 50><1 ratio |
|---|---|---|---|---|---|---|
| | | | | | | |
| | | | | | | |

| Subcutaneous | | | | | | |
|---|---|---|---|---|---|---|
| | Mouse No.1 | 300 | 0 | 0 | 0 | 0 |
| | | 100 | 0 | 0 | 0 | 0 |
| | | 33 | 0 | 0 | 0 | 0 |
| | | 11 | 0 | 0 | 0 | 0 |
| | | 4 | 0 | 0 | 0 | 1 |
| | | 1 | 0 | 0 | 0 | 0 |
| | | | | | | |
| | Mouse No.2 | 230 | 4 | 3 | 0 | 1 |
| | | 77 | 1 | 1 | 0 | 0 |
| | | 25 | 1 | 4 | 0 | 0 |
| | | 8 | 0 | 0 | 0 | 0 |
| | | 3 | 0 | 0 | 0 | 0 |
| | | 1 | 0 | 0 | 0 | 0 |

| Intraperitoneal | | | | | | |
|---|---|---|---|---|---|---|
| | Mouse No.1 | 220 | 1 | 2 | 1 | 0 |
| | | 73 | 0 | 0 | 0 | 1 |
| | | 24 | 1 | 1 | 1 | 2 |
| | | 8 | 0 | 0 | 0 | 0 |
| | | 3 | 0 | 0 | 0 | 1 |
| | | 1 | 0 | 0 | 0 | 0 |
| | | | | | | |
| | Mouse No.2 | 410 | 9 | 1 | 0 | 3 |
| | | 137 | 1 | 1 | 0 | 2 |
| | | 45 | 0 | 0 | 0 | 0 |
| | | 15 | 0 | 1 | 0 | 0 |
| | | 5 | 0 | 1 | 0 | 0 |
| | | 2 | 0 | 0 | 0 | 0 |

| Intratracheal | | | | | | |
|---|---|---|---|---|---|---|
| | Mouse No.1 | 310 | 0 | 0 | 0 | 0 |
| | | 103 | 0 | 0 | 0 | 0 |
| | | 34 | 0 | 0 | 0 | 0 |
| | | 11 | 0 | 0 | 0 | 0 |
| | | 4 | 0 | 0 | 0 | 0 |
| | | 1 | 0 | 0 | 0 | 0 |
| | | | | | | |
| | Mouse No.2 | 270 | 2 | 1 | 0 | 1 |
| | | 90 | 1 | 0 | 0 | 0 |
| | | 30 | 0 | 0 | 0 | 0 |
| | | 10 | 0 | 0 | 0 | 0 |
| | | 3 | 1 | 0 | 0 | 0 |
| | | 1 | 1 | 0 | 0 | 0 |

| Intranasal | | | | | | |
|---|---|---|---|---|---|---|
| | Mouse No.1 | 400 | 0 | 0 | 0 | 0 |
| | | 18 | 1 | 0 | 0 | 1 |
| | | 5 | 0 | 0 | 0 | 0 |
| | | 2 | 0 | 0 | 0 | 0 |
| | | | | | | |
| | Mouse No.2 | 230 | 3 | 0 | 0 | 1 |
| | | 77 | 2 | 0 | 0 | 0 |
| | | 25 | 0 | 0 | 0 | 0 |
| | | 9 | 0 | 0 | 0 | 0 |
| | | 3 | 1 | 0 | 0 | 0 |
| | | 1 | 0 | 0 | 0 | 0 |
| | | | | | | |
| | | spontaneous | 106 | 127 | 122 | 110 |
| | | maximum | 1041 | 971 | 957 | 988 |
| | | spont/max | 10 % | 13 % | 13 % | 12 % |

## Revendications

1. Composition comprenant des cellules infectées par un adénovirus recombinant défectif contenant, inséré dans son génome, un acide nucléique codant code pour tout ou partie d'un antigène spécifique d'un mélanome humain choisi parmi les protéines Mage -1, Mage-3, Bage, Rage et Gage, comprenant la partie présentée aux lymphocytes T cytotoxiques en association avec les molécules du CMH-1, capable d'induire une protection immunitaire et une destruction par le système immunitaire des cellules tumorales correspondantes.

2. Composition selon la revendication 1 **caractérisée en ce que** les cellules infectées comprennent les cellules présentatrices d'antigènes (APC).

3. Composition selon la revendication 1, **caractérisée en ce que** les cellules infectées comprennet les lymphocytes cytotoxiques spécifiques de tumeurs humaines.

4. Composition selon l'une quelconque des revendications précédentes **caractérisée en ce que** l'adénovirus comprenant, inséré dans son génome, la séquence SEQ ID n° 1.

5. Composition selon l'une quelconque des revendications précédentes **caractérisée en ce que** l'adénovirus comprenant, inséré dans son génome, la séquence comprise entre les résidus 55 à 82 de la séquence SEQ ID n°1.

6. Composition selon l'une quelconque des revendications précédentes **caractérisée en ce que** l'adénovirus comprenant, inséré dans son génome, la séquence SEQ ID n° 2.

7. Composition selon l'une quelconque des revendications précédentes **caractérisée en ce que** l'adénovirus est choisi parmi les sérotypes humains Ad2 et Ad5.

8. Composition selon l'une quelconque des revendications 1 à 6, **caractérisée en ce que** l'adénovirus est choisi parmi les sérotypes canins.

9. Composition selon l'une quelconque des revendications précédentes **caractérisée en ce que** l'adénovirus comporte une délétion dans la région E1.

10. Composition selon l'une quelconque des revendications précédentes **caractérisée en ce que** l'adénovirus comporte en outre une délétion dans la région E4.

11. Composition selon l'une quelconque des revendications précédentes **caractérisée en ce que** l'acide nucléique est inséré dans la région E1 ou E3 ou E4.

## Patentansprüche

1. Verbindung mit durch einen mit in seinem Genom eingefügter, ganz oder teilweise einen Code eines spezifischen Antigens eines aus den Proteinen Mage -1, Mage-3, Bage, Rage und Gage ausgewählten menschlichen Melanoms kodierenden Nukleinsäure defektiven rekombinanten Adenovirus infizierten Zellen, mit dem den zytotoxischen Lymphozyten T in Verbindung mit den Molekülen des CMH-I präsentierten Teil, die zur Induktion eines Immunschutzes und einer Zerstörung der entsprechenden Tumorzellen durch das Immunsystem fähig ist.

2. Verbindung gemäß Anspruch 1, **dadurch gekennzeichnet, dass** die infizierten Zellen Antigene aufweisende Zellen (APC) umfassen.

3. Verbindung gemäß Anspruch 1, **dadurch gekennzeichnet, dass** die infizierten Zellen für menschliche Tumoren spezifische zytotoxische Lymphozyten umfassen.

4. Verbindung gemäß einem der vorherigen Ansprüche, **dadurch gekennzeichnet, dass** der Adenovirus die Sequenz SEQ ID Nr. 1 in seinem Genom eingefügt umfasst.

5. Verbindung gemäß einem der vorherigen Ansprüche, **dadurch gekennzeichnet, dass** der Adenovirus die zwischen den Resten 55 bis 82 der Sequenz SEQ ID Nr. 1 inbegriffene Sequenz eingefügt in seinem Genom umfasst.

6. Verbindung gemäß einem der vorherigen Ansprüche, **dadurch gekennzeichnet, dass** der Adenovirus die Sequenz SEQ ID Nr. 2 eingefügt in seinem Genom umfasst.

7. Verbindung gemäß einem der vorherigen Ansprüche, **dadurch gekennzeichnet, dass** der Adenovirus aus den menschlichen Serotypen Ad2 und Ad5 ausgewählt ist.

8. Verbindung gemäß Anspruch 1 bis 6, **dadurch gekennzeichnet, dass** der Adenovirus aus den Hunde-Serotypen ausgewählt ist.

9. Verbindung gemäß einem der vorherigen Ansprüche, **dadurch gekennzeichnet, dass** der Adenovirus eine Defizienz in der Region E1 umfasst.

10. Verbindung gemäß einem der vorherigen Ansprüche, **dadurch gekennzeichnet, dass** der Adenovirus darüber hinaus eine Defizienz in der Region E4 umfasst.

11. Verbindung gemäß einem der vorherigen Ansprüche, **dadurch gekennzeichnet, dass** die Nukleinsäure in der Region E1 oder E3 oder E4 eingefügt ist.

## Claims

1. Composition comprising cells infected by a defective recombinant adenovirus containing in its genome, a nucleic acid coding code for all or part of a specific antigen of a human melanoma chosen from among the proteins Mage-1, Mage-3, Bage, Rage and Gage, comprising the part presented to the cytotoxic T-lymphocytes in association with the molecules of CMH-1, able to induce immune protection and destruction by the immune system of the corresponding tumoral cells.

2. Composition according to claim 1, **characterised in that** the infected cells contain the antigen presenting cells (APC).

3. Composition according to claim 1, **characterised in that** the infected cells contain the specific cytotoxic lymphocytes of human tumours.

4. Composition according to any of the previous claims, **characterised in that** the adenovirus, inserted in its genome, contains the sequence SEQ ID n° 1.

5. Composition according to any of the previous claims, **characterised in that** the adenovirus, inserted in its genome, contains the sequence between residues 55 to 82 of the sequence SEQ ID n° 1.

6. Composition according to any of the previous claims, **characterised in that** the adenovirus, inserted in its genome, contains the sequence SEQ ID n° 2.

7. Composition according to any of the previous claims, **characterised in that** the adenovirus is chosen from among human serotypes Ad2 and Ad5.

8. Composition according to any one of claims 1 to 6, **characterised in that** the adenovirus is chosen from among the canine serotypes.

9. Composition according to any of the previous claims, **characterised in that** the adenovirus comprises a deletion in region E1.

10. Composition according to any of the previous claims, **characterised in that** the adenovirus also comprises a deletion in region E4.

11. Composition according to any of the previous claims, **characterised in that** the nucleic acid is inserted in region E1 or E3 or E4.
